Europäisches Patentamt

(19) European Patent Office

Office européen des brevets

(11) Publication number: **0 073 134**
**A2**

# (12) EUROPEAN PATENT APPLICATION

(21) Application number: **82304342.7**

(22) Date of filing: **17.08.82**

(51) Int. Cl.³: **C 12 P 7/66**
**C 12 N 1/20**
**//C12N1/32, C12N1/36, C12R1/01**

(30) Priority: **19.08.81 GB 8125257**

(43) Date of publication of application:
**02.03.83 Bulletin 83/9**

(84) Designated Contracting States:
**AT BE CH DE FR GB IT LI LU NL SE**

(71) Applicant: **Pfizer Limited**
**Ramsgate Road**
**Sandwich Kent CT13 9NJ(GB)**

(84) Designated Contracting States:
**GB**

(71) Applicant: **Pfizer Corporation**
**Calle 15 1/2 Avenida Santa Isabel**
**Colon(PA)**

(84) Designated Contracting States:
**BE CH DE FR IT LI LU NL SE AT**

(72) Inventor: **Hall, Michael Christopher**
**Broadacres Stodmarsh**
**Nr. Canterbury Kent(GB)**

(74) Representative: **Moore, James William**
**Pfizer Limited Ramsgate Road**
**Sandwich Kent CT13 9NJ(GB)**

(54) Microbiological process for the preparation of hydroquinone.

(57) Hydroquinone is prepared by contacting benzene with a culture of a methylotrophic microorganism grown on methanol as sole energy and carbon source.

This invention relates to a microbiological transformation process for the preparation of hydroquinone from benzene using methylotrophic microorganisms.

Methylotrophic organisms are methane-utilising organisms, which can use single-carbon compounds as their principal source of carbon and energy. These organisms fall into three main classes: the first type comprises the obligate methylotrophic bacteria that will only grow on single-carbon compounds as their energy and carbon source; the second type are the facultative methylotrophic bacteria which can grow on single-carbon compounds, but are also capable of growing on other organic compounds such as glucose; while the third type are certain methane-utilising yeasts.

The obligate methylotrophic bacteria can be further divided into two types on the basis of their morphology and the type of assimilation pathway used. Type I organisms, which includes Methylomonas and Methylococcus species have their membranes dispersed throughout the cell and use the hexulose monophosphate pathway of assimilation. Included in this group are Methylococcus capsulatus, Methylomonas methanica and Methylomonas albus.

Type II organisms, such as Methylosinus species, have their membranes arranged in peripheral fashion within the cell and assimilate their carbon from the serine pathway. An example of this type is Methylosinus trichosporium.

The enzyme methane mono-oxygenase present in all these species of bacteria is responsible for the initial oxidation of methane to yield methanol. This enzyme has also been shown to be able to insert an atom of oxygen into a variety of hydrocarbon substrates, including alkanes, alkenes and various aromatic compounds. Hitherto these organisms have been cultured on methane as the sole energy source for this purpose, and the biotransformation reactions observed generally involve the insertion of a single atom of oxygen into the substrate molecule. Thus U.K. patent application serial number 2024205A describes a number of biotransformation reactions using cells of Methylosinus trichosporium grown in an atmosphere containing methane. In particular the oxidation of benzene to yield phenol is described and also the oxidation of phenol to yield a mixture of catechol and 1,4-dihydroxybenzene (hydroquinone).

It has now surprisingly been discovered that, when the organism is grown on methanol as the sole energy and carbon source and the cells grown in this way are used, it is possible to oxidise benzene directly and cleanly in a single step to give hydroquinone as the sole product. Hydroquinone (1,4-dihydroxybenzene) is a valuable product of particular use in the photographic industry as a reducer and developer.

Thus according to the present invention there is provided a single-step microbiological transformation process for the preparation of hydroquinone from benzene. In particular the invention provides a process for the preparation of hydroquinone which comprises contacting benzene with cells of a methylotrophic microorganism grown on methanol as the sole energy and carbon source, or a preparation or extract thereof containing an oxidative enzyme system, and recovering the produced hydroquinone.

A particularly suitable microorganism for use in the process is the known strain Methylosinus trichosporium OB3b deposited at

the National Collection of Industrial Bacteria, Torry Research Station, Aberdeen, Scotland as NCIB 11131.

Thus the invention also provides bacteria from the strain Methylosinus trichosporium OB3b adapted to grow on methanol as the sole energy and carbon source by cultivation in a culture medium to which liquid methanol is added as the only carbon and energy source.

Before use in the process of the invention the methylotrophic organism must be grown using methanol as the carbon source.

Growing the organism on methanol has a number of advantages for large scale or industrial use of the process. Thus it avoids the hazards and difficulties associated with the use of methane gas, methanol being easier to contain and less of a fire risk. In addition it is simpler to monitor the concentration of methanol in the culture broth, for example by using the standard analytical techniques of gas liquid chromatography, also, since the methanol is entirely dissolved in the liquid nutrient medium, there is much better contact with the growing cells. The organism can be grown under batch or continuous conditions and these conditions and possible variations thereof appropriate to the particular microorganism strain will be well known to those skilled in the art.

In commercial processes for the propagation of microorganisms it is generally necessary to proceed by stages. These stages may be few or many depending on the nature and scale of the process, and the characteristics of the microorganism. Ordinarily propagation is started by inoculating cells into a pre-sterilised nutrient medium usually contained in a flask. In the flask the growth of the microorganism is encouraged by various means, for example by maintaining a suitable temperature and by shaking for thorough aeration. This step may be repeated one or more times in flasks or vessels containing larger volumes of nutrient medium. The microorganisms from the last development stage, with or without the accompanying culture medium, are introduced into a large scale fermenter to produce the desired quantities of the microorganism. To obtain maximum yields of the microbial cells the conditions during culture and fermentation need to be carefully controlled. A temperature of between 25 and 35°C is generally employed, a temperature of 28 to 30°C being preferred. The pH is also carefully controlled in the range 6.0 to 7.5, preferably 6.6 to 7.2, a pH of 6.8 being optimal. The pH is conveniently controlled by adding nitric acid and this is most conveniently done using a pH⁻ stat. The culture medium used for growing the microorganism contains nitrate, phosphate and sulphate salts as well as calcium, iron (as its ethylenediaminetetraacetic acid complex) trace elements and is sterilised before use. The source of oxygen for the culture is generally air. The methanol required for growth is added directly to the fermention in portions or continuously, during the fermentation, it being best to avoid exceeding concentrations of more than 2% methanol by volume in the medium. The cells from the cultures are harvested during the late logarithmic phase and this may be done by standard techniques, for example by flocculation, sedimentation or centrifugation and the cells are washed with water. Alternatively the broth may be used directly for the biotransformation process and, in this case, fermentation is continued to allow all the methanol to be consumed before use. For continuous flow culture the microorganism may be grown in any suitably adapted fermentation vessel, for example a stirred baffled fermenter which is provided either with internal cooling or an external recycle cooling loop. Fresh medium and methanol may be continuously pumped into the culture and the culture removed at such a rate that the volume of culture and cell density remain constant.

Rather surprisingly, even though cells grown on methanol do not require the enzyme methane mono-oxygenase (which is responsible for the initial oxidative attack to convert methane to methanol), it is believed that this enzyme is nevertheless produced and is in a highly active form. This is thought to be responsible for enabling the subsequent benzene biotransformation process to be performed, although the nature of the enzyme systems involved is not fully understood.

Although a suspension of whole cells is most conveniently used in the process of the invention, it is also possible to use ruptured cells or a partially purified crude extract or cell-free particulate fraction containing the oxidative enzyme system. In addition various processes have been described for preparing immobilised cell preparations or immobilised enzyme extracts, for example by fixing the cells or enzyme in an insoluble polysaccharide matrix. These possibilities and methods and conditions for their performance will be well known to those skilled in the art.

To put the process of the invention into practice, benzene is contacted with a culture of whole cells of the microorganism grown on methanol as described above or using an extract or preparation thereof containing an oxidative enzyme system, and the mixture is incubated until the biotransformation is complete. The hydroquinone is then recovered by conventional means.

In a typical biotransformation, benzene is added to a crude fermentation broth containing cells of _Methylosinus trichosporium_ OB3b prepared by growing on methanol as described above. For best results broths containing a cell density in the range 5 to 60 g/litre are preferred and in particular a cell density of 10-20 gm of dry cells per litre. Benzene is added in an amount generally in the range 0.1 to 10 ml/litre of broth. The mixture is incubated at a temperature in the range 25 to 35$^{o}$C, a temperature of 28 to 30$^{o}$C being preferred and the pH of the culture is maintained in the range 6.8 to 7.2, a pH of 7.0 being ideal. Under these conditions the biotransformation process proceeds rapidly and is generally substantially complete within a period of 2 to 5 hours, depending on the quantities of cells present and benzene added. Progress of the reaction can be monitored using gas chromatography and the process is terminated when all the benzene has been converted to hydroquinone. The produced hydroquinone is recovered by conventional techniques, for example by solvent extraction using a water-immiscible organic solvent. Ethyl acetate is a suitable solvent; drying and evaporation of the extract yield hydroquinone in a state of high purity.

It will be apparent that the quantity of benzene which can be oxidised by a given quantity of cells is limited, however it is possible to extend or regenerate the oxidising power of the cells by adding an electron source, for example using sodium formate, formaldehyde or methanol. This may either be added during the biotransformation or the used cells may be regenerated in a separate step, for example by suspending the separated cells in a solution of sodium formate overnight. The cells may then be re-used several times in the biotransformation process.

As an alternative a continuous process may be used in which benzene is continuously added to the culture of the cells and the produced hydroquinone is constantly removed. Appropriate monitoring of pH and other process parameters will be required and the cells are replenished as necessary to maintain a viable culture, however, these requirements are entirely within the knowledge and skill of those familiar with the art.

The following examples illustrate growth of methylotrophic organisms on methanol and their use in the process for the preparation of hydroquinone from benzene.

EXAMPLE 1

Growth of Methylosinus trichosporium on methanol

(1)  Media

The incubation medium was prepared according to Whittenbury et. al. J. Gen. Microbiol., 61, 205-218, 1970 in adapted form. Solutions were made up as follows:

(a)  Trace elements solution

| | |
|---|---|
| $Na_2EDTA$ | 500 mg |
| $FeSO_4.7H_2O$ | 200 mg |
| $ZnSO_4.7H_2O$ | 10 mg |
| $MnCl_2.4H_2O$ | 3 mg |
| $H_3BO_3$ | 30 mg |
| $CoCl_2.6H_2O$ | 20 mg |
| $CuCl_2.2H_2O$ | 1 mg |

| | |
|---|---|
| $NiCl_2.6H_2O$ | 2 mg |
| $NaMoO_4.2H_2O$ | 3 mg |
| distilled water to | 1 litre |

(b)  FeEDTA solution

| | |
|---|---|
| FeEDTA | 8 g |
| distilled water to | 1 litre |

(c)  Phosphate buffer

| | |
|---|---|
| $Na_2HPO_4$ | 49.7 g |
| $KH_2PO_4$ | 39.0 g |
| distilled water to | 1 litre |

The following ingredients were dissolved in distilled water to give a final volume of 50 litres and sterilised by autoclaving at $121^{o}$C for 15 minutes:

| | |
|---|---|
| $CaCl_2.2H_2O$ | 20 g |
| $KNO_3$ | 100 g |
| $MgSO_4.7H_2O$ | 100 g |
| FeEDTA solution | 50 ml |
| Trace elements solution | 50 ml |
| Ucon antifoam | 5 ml |

After the sterilised medium was cooled, 500 ml of AR methanol (sterilised by filtration through a 0.45 micron pore size filter) and 1 litre of sterile phosphate buffer was added aseptically.

(2)  Inoculum

5 Ml of frozen <u>Methylosinus trichosporium</u> OB3b culture was inoculated into a sterile 300 ml Erlenmayer flask containing 100 ml of the above medium.  It was incubated at 28$^{\circ}$C on a rotary shaker (220 rpm) for 72 hours.  10 ml aliquots of this culture were used to inoculate 300 ml Erlenmayer flasks containing 100 ml of the above medium.  These were incubated at 28$^{\circ}$C on a rotary shaker (220 rpm) for 48 hours.  The contents of each flask were then used to inoculate 2.8 litre Fernbach flasks containing 1 litre of the above medium.  The flasks were incubated at 28$^{\circ}$C on a rotary shaker (220 rpm) for 48 hours.

(3)  Fermentation

A 75 litre fermenter containing 50 litres of medium prepared as described above was inoculated aseptically with the contents of 5 Fernbach flasks prepared as described above.  The culture was aerated with 25 litres/minute of air, and agitated at 450 rpm. The pH of the culture was maintained at 6.8 by the automatic addition of a feed of the following composition:

| | |
|---|---|
| concentrated nitric acid | 360 ml |
| FeEDTA solution | 380 ml |
| Trace elements solution | 600 ml |
| distilled water | 2820 ml |
| A.R. methanol | 5840 ml |

This feed was prepared in a sterile form by autoclaving all ingredients except methanol (which was filtered sterilised and added aseptically) at 121°C for 30 minutes. Sterile methanol was added after the other ingredients had cooled to room temperature. After 56.5 hours this feed was replaced by a solution of nitric acid prepared as follows:

concentrated nitric acid      75 ml

distilled water to           500 ml

autoclaved at 121° for 15 minutes

This solution was fed automatically to maintain culture pH at 6.8 while 1.722 litres of filter sterilised methanol was added aseptically to the culture at the same time. Incubation of the culture was continued until 81 hours after inoculation, at which time all the methanol had been consumed.

55 Litres of culture broth containing 16.1 g dry cells/litre was obtained; this was used directly for biotransformations without further treatment.

EXAMPLE 2

Growth of Methylosinus trichosporium on methanol

200 Ml of shake flask grown culture of Methylosinus trichosporium prepared as described in Example 1(2) was inoculated in 2 litres of medium (prepared as in Example 1(1) but in this case containing half the quantities of FeEDTA and trace elements) in a 3 litre fermentation vessel. The fermenter was aerated at 750 ml/minute and agitated at 1000 rpm.  Temperature was maintained at 28°C and the pH was controlled at 6.8 by the automatic addition of a feed of the following composition:

| Concentrated nitric acid | 30.9 ml |
|---|---|
| FeEDTA solution | 18.8 ml |
| Trace elements solution | 30.0 ml |
| distilled water | 195.3 ml |
| Methanol A.R. | 225 ml |

After 52 hours fermentation 200 ml sterile methanol was added, and after 58 hours a further 100 ml of sterile methanol was added.  Aeration was increased to 1000 ml/minute and agitation was increased to 1300 rpm. The fermentation was terminated after 69.5 hours; the broth contained 14.0 g dry cells/litre and less than 0.01% methanol.

## EXAMPLE 3

### Preparation of hydroquinone

Benzene (2 ml reagent grade) was added in one lot to two litres of the broth obtained in Example 1, containing 16.1 g/litre of whole cells of Methylosinus trichosporium OB3b in a chemostat (L. H. Engineering Ltd.). The mixture was incubated for 2.5 hours with the temperature maintained at 30°C, the pH controlled at 6.9 and a stirring speed of 1000 rpm with aeration at 200 cc per minute.  An aliquot of the aqueous phase was removed and extracted with ethyl acetate.  Gas chromatographic analysis of the extract indicated the presence of hydroquinone with no traces of benzene or phenol.  A further 2 ml benzene was added to the culture medium and the incubation continued for a further 2.5 hours.  The broth was then extracted with ethyl acetate (600 ml) and the extract dried (MgSO$_4$) and evaporated to yield a pinkish solid (2.8 g) whose nuclear magnetic resonance and infrared spectra were identical to an authentic sample of hydroquinone. Gas chromatographic analysis indicated that the product was better than 99% pure.

## EXAMPLE 4

### Preparation of Hydroquinone

Benzene (200 $\mu$l) was incubated at 30$^\circ$C on a rotary shaker at 300 rpm for 5 hours with 800 cc of a broth containing 8.8 g/litre of whole cells of M. trichosporium OB3b grown on methanol as described in Example 2. The whole broth was extracted with ethyl acetate (5 x 100 ml) and the extracts combined, dried and evaporated to yield 150 mg hydroquinone.

## EXAMPLE 5

### Preparation of hydroquinone

A 300 ml shake flask containing 20 ml of a whole cell culture of Methylosinus trichosporium OB3b grown on methanol (13.3 g/litre) was incubated with 20 $\mu$l (0.016 g) of benzene for four hours at 30$^\circ$C on a rotary shaker. Gas chromatographic analysis showed that no benzene remained and only hydroquinone was present. The whole broth was extracted with ethyl acetate (6 ml) which was evaporated to yield 16 mg hydroquinone.

## EXAMPLE 6

### Preparation of hydroquinone using regenerated cells

A broth (500 ml) containing 18.0 g/litre of cells of Methylosinus trichosporium OB3b grown on methanol was incubated with 0.5 ml of benzene at 30$^\circ$C for 5 hours on a rotary shaker. The cells were removed by centrifugation. Analysis of the supernatant indicated the presence of hydroquinone. One half of the centrifuged cells were resuspended in water (250 ml) containing sodium formate and the suspension stirred overnight at $4^{o}$C. Reincubation of the cells with a further 0.25 ml benzene at $30^{o}$C for 5 hours produced more hydroquinone.

The procedure could be repeated for at least eight regenerations while cells that were not treated with sodium formate lost activity after two cycles.

CLAIMS

1. A process for the preparation of hydroquinone which comprises contacting benzene with cells of a methylotrophic microorganism grown on methanol as the sole energy and carbon source, or a preparation or extract thereof containing an oxidative enzyme system, and recovering the produced hydroquinone.

2. A process according to claim 1 wherein the methylotrophic microorganism is Methylosinus trichosporium OB3b.

3. A process according to claim 1 or claim 2 wherein the benzene is contacted with a suspension of whole cells of the methylotrophic microorganism.

4. A process according to claim 3 wherein the suspension is a crude fermentation broth.

5. A process according to claim 3 or claim 4 wherein the culture is maintained at a temperature of from 28 to 30°C and a pH in the range 6.8 to 7.2.

6. A process according to any one of claims 3 to 5 wherein the cell density is from 5 to 60 gm of dry cells per litre.

7. A process according to any one of claims 3 to 6 wherein sodium formate, formaldehyde or methanol is added as an electron source.

8. A process according to any one of claims 1 to 7 wherein the produced hydroquinone is recovered by extraction with a water-immiscible organic solvent.

9. A single step biotransformation process for the preparation of hydroquinone from benzene substantially as hereinbefore described with particular reference to Examples 3 to 6.

10.  Bacteria adapted from the strain Methylosinus trichosporium OB3b to grow on methanol as the sole energy and carbon source by cultivation in a culture medium to which liquid methanol is added as the only carbon and energy source.